# EUROPÄISCHE PATENTSCHRIFT

(11) **EP 0 930 238 B1**
(45) Veröffentlichungstag und Bekanntmachung des Hinweises auf die Patenterteilung: **22.08.2001**
(21) Anmeldenummer: 98123979.1
(22) Anmeldetag: 17.12.1998
(51) Int. Cl.: B65D 1/09, A61J 1/06

(54) **Befüllter und verschlossener Kunststoffbehälter und Verfahren zu seiner Herstellung**
Filled and closed plastic container, and process for its manufacture
Récipient en plastique rempli et fermé, et procédé pour sa fabrication

(30) Priorität: 16.01.1998 DE 19801320
(43) Veröffentlichungstag der Anmeldung: 21.07.1999
(73) Patentinhaber: Schott Glas, 55122 Mainz (DE); CARL-ZEISS-STIFTUNG trading as SCHOTT GLASWERKE, 55122 Mainz (DE)
(72) Erfinder: Walther, Marten Dr., 55270 Engelstadt (DE); Spallek, Michael Dr., 55218 Ingelheim (DE); Geiger, Andreas, 32257 Bünde (DE)
(74) Vertreter: Fuchs Mehler Weiss & Fritzsche

(56) Entgegenhaltungen:
- EP-A- 0 709 485
- EP-A- 0 787 823
- DE-A- 3 833 036

## Beschreibung

Die Erfindung bezieht sich auf einen befüllten und verschlossenen Kunststoffbehälter, ausgeformt nach der Blasform- oder Spritzgießtechnik, und in-line befüllt sowie verschlossen, der mindestens eine Sperrschicht gegen Gase, Wasserdampf sowie organische Moleküle aufweist.

Die Erfindung betrifft ferner ein Verfahren zur Herstellung eines derartigen befüllten und verschlossenen Kunststoffbehälters.

Kunststoffbehälter dieser Art werden typischerweise für medizinische Zwecke oder zur Verpackung von Getränken bzw. flüssigen Nahrungsmitteln verwendet.

So haben insbesondere Kunststoffbehälter für medizinische Zwecke als Pharmaverpackung eine große Verbreitung gefunden. Sie können relativ einfach, z.B. durch Extrusionsblasen, wenn der Behälter nicht starr sein muß, oder im Fall von starren Behältern nach der Spritzgießtechnik oder Spritzblastechnik hergestellt werden. Die typischen Kunststoff-Formgebungsverfahren gehen beispielsweise aus dem Buch von Helbig-Spingler, "Kunststoffe für die Pharmazeutische Verpackung", Wissenschaftliche Verlagsgesellschaft mbH Stuttgart 1985 hervor.

Allerdings sind der Anwendung der vorgenannten Kunststoffbehälter Grenzen gesetzt. Eine dieser Grenzen ist die begrenzte Sperreigenschaft der Kunststoffbehälter-Wandung gegen Gase bzw. Dämpfe, insbesondere gegen Sauerstoff, Kohlendioxid, Wasserdampf, und Lösungsmittel, z.B. aus dem Etikettenklebstoff, die die Lagerzeit der befüllten Kunststoffbehälter begrenzt. Das macht sich insbesondere bei kleinvolumigen Pharmaverpackungen (z.B. kleinen vorgefüllten Spritzen) mit einem großen Verhältnis von Behältnisoberfläche zu dessen Füllvolumen nachteilig bemerkbar.

Es ist bekannt, diesem Problem dadurch gerecht werden zu wollen, daß man spezielle Kunststoffe mit hohen Sperreigenschaften verwendet. In den meisten Fällen bilden jedoch derartige spezielle Kunststoffe nur gegen bestimmte Gase, bzw. Dämpfe eine Sperre, wodurch mehrschichtige Behälter mit unterschiedlichen Kunststoffen angefertigt werden mußten, was aus anderen, insbesondere fertigungstechnischen Gründen, wiederum nachteilig ist.

Am meisten verbreitet ist daher die Methode, an dem Kunststoffbehälter selbst eine Sperrschicht auszubilden, wodurch man auch in der Wahl des Kunststoffes für den Behälter selbst, insbesondere hinsichtlich seiner mechanischen und optischen Eigenschaften, freier ist.

Um eine Barriere-Schicht gegen Lösungsmittel zu bilden, ist es bekannt, die Oberfläche von PE-Blaskörpern chemisch zu modifizieren, insbesondere durch Sulfonierung oder Fluorierung (VDI-Buch "Sperrschichtausbildung bei Kunststoff-Hohlkörpern", VDI-Verlag, Düsseldorf 1986, S. 97-107). Diese Methode ist jedoch speziell auf die Sperre gegen organische Lösungsmittel und nicht gegen Gase bzw. Wasserdampf abgestellt.

Auch ist das Aufbringen von Schutzschichten durch Plasmapolymerisation bzw. plasmainduzierte Polymerisation bekannt (Bosch Technische Berichte 8 (1986/87) 5, S. 219-226). Hierbei geht es jedoch mehr um einen Schutz gegen äußere, insbesondere mechanische Einwirkungen.

Um eine breite Sperrwirkung gegen Gase, Wasserdampf und organische Lösungsmittel zu erhalten, ist es bekannt, den Kunststoffbehälter mit einer Beschichtung aus speziellen organischen und anorganischen Materialien zu versehen.

So ist durch den Aufsatz: "Multilayer Barrier Coating System produced by Plasma-impulse Chemical Vapor Deposition (PRCVD)", von M. Walther, M. Heming, M. Spallek, veröffentlicht in "Surface and Coatings Technology" 80 (1996), S. 200-205 bekannt geworden, fertig ausgeformte Kunststoffbehälter mit einer SiOₓC_{y}H_{z}- bzw. TiOₓC_{y}H_{z}-Schicht zu versehen. Dabei erfolgt die Beschichtung nach dem PICVD-Verfahren (Plasma-Impuls-Chemical-Vapor-Deposition), welches z.B. durch die DE 40 08 405 C1 sowie durch die US-A-5,154,943 bekannt geworden ist.

Durch die DE 44 38 359 A1 ist ferner ein Kunststoffbehälter bekannt geworden, der mit einer Sperrschicht versehen ist, die aus sequentiell angeordneten Sperrschichten aus organischen Polymeren und aus anorganischen Oxiden, Nitriden oder Oxinitriden besteht. Beschichtet wird auch in diesem Fall der ausgeformte Kunststoffbehälter auf der Innenseite nach dem PICVD-Verfahren, wie es insbesondere in der DE 196 29 877 beschrieben wird.

In allen bekannten Fällen, in denen ein Kunststoffbehälter durch Extrusionsblasen oder Spritzgießen hergestellt wird, wird die Sperrschicht typischerweise als Innenschicht im Behälter aufgebracht. Die Aufbringung der Innenschicht erfolgt dabei nach abgeschlossener Ausformung des notwendigerweise noch unbefüllten Kunststoffbehälters in einer nachgeschalteten Beschichtungsstation, mit einer Untervariante, wie sie durch die DE 195 02 103 bekannt geworden ist, gemäß der das Blasformgas zugleich das Prozeßgas für die Beschichtung ist.

Auch ist es durch das vorgenannte VDI-Buch bekannt (Seite 85), beim Streckblasen den Vorformling alternativ außen oder innen zu beschichten. Auch in diesem Fall ist der vorgeformte Behälter ungefüllt.

Die Schichtmaterialien sind dabei stets so gewählt, daß sie ein breites Spektrum abdecken, d.h. behälterinhaltsunspezifisch sind, da die Beschichtung an unbefüllten Kunststoffbehältern vorgenommen werden muß, und man zu diesem Zeitpunkt nicht weiß, welche Füllung die Kunststoffbehälter später erhalten, es sei denn, es würden spezielle Chargen gebildet, was den Herstellungsprozeß jedoch erheblich verkomplizieren würde. Diese Breitspektrum-Sperrschichten können jedoch typischerweise nicht allen speziellen Inhaltsstoffen optimal gerecht werden.

Durch eine aufkommende In-Line-Fertigung von befüllten und verschlossenen Kunststoffbehältern sind die Schritte des Ausformens des Kunststoffbehälters sowie seine Befüllung und sein Verschließen sozusagen näher gerückt. Diese In-Line-Fertigung für die Extrusionsblastechnik - unter dem Schlagwort "blow-fill-seal" bekannt, ist z.B. durch die DE 38 33 036, die DE 38 23 428 und die US-Patentschriften 4 671 763, 3 919 374 und 4 995 511, bekannt geworden. Die In-Line-Fertigung für die Spritzgießtechnik - unter dem Schlagwort "injection mold-fill-seal" bekannt, ist in der älteren Patentanmeldung DE 196 52 708.2-35 beschrieben.

Trotz des Näherrückens der Fertigungsschritte erfolgt das Aufbringen der Sperrschicht auf den Kunststoffbehälter weiterhin inhaltsunspezifisch und typischerweise als Innenschicht mit den vorbeschriebenen Nachteilen.

Der Erfindung liegt die Aufgabe zugrunde, Kunststoffbehälter der eingangs genannten Art mit einer inhaltsspezifischen Sperrwirkung zu schaffen.

Die Lösung dieser Aufgabe gelingt Erfindung ausgehend von dem eingangs bezeichneten befüllten und verschlossenen Kunststoffbehälter, sowie ausgeformt nach der Extrusionsblas-, Spritzblas- oder Spritzgießtechnik und in-line befüllt sowie verschlossen, der zumindestens eine Sperrschicht gegen Gase, Wasserdampf und organische Moleküle aufweist, gemäß der Erfindung dadurch, daß die Sperrschicht aus einem behälterinhaltsspezifischen Material besteht und an der Außenseite des befüllten Kunststoffbehälters, einschließlich dessen Verschlusses, nach seiner Befüllung aufgebracht ist.

Da zum Zeitpunkt der Beschichtung die Behälter befüllt sind, d.h. der Inhaltsstoff bekannt ist, kann ohne weiteres eine inhaltsspezifische Beschichtung vorgesehen werden.

Die Sperrschicht hat keine Berührung mit dem Inhalt des Behälters und kann somit nicht mit dem Behälterinhalt in Wechselwirkung treten. Dies erlaubt es, das Material des Kunststoffbehälters speziell auf die Inhaltsstoffe und die Materialien für die Sperrschicht speziell auf die Sperrschichtwirkung abzustellen. Bislang mußten beim Befüllen der innenbeschichteten Kunststoffbehälter bei einem Wechsel der Inhaltsstoffe vorab entsprechende Stabilitätsuntersuchungen durchgeführt werden, die bei den gemäß der Erfindung hergestellten Kunststoffbehältern entfallen.

Die durch das Beschichten der Außenseite des Behälters nach dem Befüllen gewonnenen Freiheitsgrade in der Wahl der Schichtmaterialien unabhängig von der Wahl des Kunststoffmaterials für den Behälter, ermöglichen es auch, daß die Schichtstoffe farbig/trüb sein können, z.B. um Lichtschutz zu gewährleisten oder Farbkodierungen vorzunehmen. Insbesondere ist ein UV-Schutz des Behälters möglich, wodurch mit Vorteil keine oder jedenfalls nur wenige Additive im Kunststoff, aus dem die Behälter gefertigt werden, nötig sind.

Da der befüllte Kunststoffbehälter ferner typischerweise, wenn es das eingefüllte Präparat zuläßt, einer Terminalsterilisation unterworfen wird, in der Regel durch Autoklavieren bei 121° für eine Dauer von 20 Minuten, ist die Außen-Sperrschicht, wenn sie nach dem Sterilisationsvorgang aufgebracht wird, der Belastung durch diesen nachhaltigen Verfahrensschritt nicht ausgesetzt, was ebenfalls Freiheitsgrade bei der Wahl der Schichtmaterialien schafft, Freiheitsgrade, die bei einer Innenbeschichtung von in-line hergestellten Behältern nicht gegeben sind, da dort sowohl auf die Substanzverträglichkeit als auch auf die Sterilisierbarkeit Rücksicht genommen werden muß.

Da typischerweise die für die Sperrschicht verwendeten Materialien einen besseren Haftgrund als die für den Behälter verwendeten Kunststoffe abgeben, wird auch eine erhöhte Haftfestigkeit von Etiketten bzw. von Bedruckungsfarben gewährleistet, die typischerweise erst nach dem Beschichten aufgebracht werden. Es entfallen dadurch mit Vorteil vorbereitende Schritte vor der Bedruckung. Die Außenbeschichtung verhindert dabei mit großem Vorteil eine Migration von Druckfarbenlösungsmitteln oder von Etikettenkleberbestandteilen in das Füllgut.

Die Außenbeschichtung der Behälter erlaubt es auch, daß nicht nur einzelne Behälter, sondern ganze Blocks oder Streifen (typischerweise ca. 5 - 10 miteinander verbundene Einzelbehältnisse) beschichtet werden können.

Die Beschichtung beeinflußt nicht den Verschlußvorgang, das Zuschmelzen, beschichtet jedoch den Verschluß mit und sorgt somit für eine geschlossene Sperrschicht. Eine derartige Mitbeschichtung des Verschlusses ist bei einer Innenbeschichtung praktisch nicht möglich.

Es ist bei coextrudierten Flaschen bekannt (VDI-Buch a.a.O., S. 61), bei der Herstellung von Flaschen außen eine Sperrschicht durch einen Satellitenextruder anzubringen. Bei diesem Verfahren ist die Sperrschicht jedoch nur bei unbefüllten Behältern und nur in relativ dicken Schichten, die z.B. für Pharmaverpackungen nicht geeignet sind, aufbringbar. Ferner können nur Materialien verwendet werden, die extrudierbar sind, so daß nur eine begrenzte Einsatzmöglichkeit von Sperrschichtstoffen gegeben ist. Dieses bekannte Verfahren ist daher nur für ganz spezielle Fälle im Bereich der Agrochemie, der Kosmetik und der Lebensmittelverpackungen, speziell für Tomaten-Ketchup, geeignet.

Gemäß einer Weiterbildung der Erfindung ist der Behälter vorzugsweise nach dem sogenannten "Blow-Fill-Seal-Verfahren" hergestellt, mit dem sich auf einfache Weise ein nicht starrer Behälter herstellen läßt, der, wie noch anhand des Verfahrens zu beschreiben sein wird, für die nachträgliche Beschichtung mit einer Sperrschicht besonders vorteilhafte Eigenschaften hat.

Der Erfindung liegt ferner die Aufgabe zugrunde, ein vorteilhaftes Verfahren zur Herstellung solcher befüllter, verschlossener und beschichteter Kunststoffbehälter zu schaffen.
Die Lösung dieser Aufgabe gelingt gemäß der Erfindung mit den in einer geschlossenen Fertigungslinie ausgeübten Schritten:
- Ausformen des Kunststoffbehälters,
- Befüllen des Kunststoffbehälters,
- Verschließen des Kunststoffbehälters,
- optional Sterilisieren des verschlossenen Kunststoffbehälters
- Beschichten der Außenseite des Kunststoffbehälters einschließlich seines Verschlusses mit einer behälterinhaltsspezifischen Sperrschicht, und
- optional Sterilisieren des beschichteten Kunststoffbehälters.
Gemäß einer Weiterbildung der Erfindung erfolgt die Aufbringung der Außenschicht auf den verschlossenen Kunststoffbehälter auf die vom Ausformen noch warme Außenhaut nach einem Vakuumverfahren, wodurch sich mit Vorteil eine hohe Haftung der Sperrschicht auf der Außenseite des Behälters erzielen läßt.

Weitere ausgestaltende Merkmale und Vorteile der Erfindung ergeben sich anhand von in den Zeichnungen dargestellten Ausführungsbeispielen.

Es zeigen:
- Fig. 1: einen gemäß der Erfindung ausgebildeten, befüllten und verschlossenen Kunststoffbehälter in Form einer Ampulle in einer Frontansicht,
- Fig. 2: den Kunststoffbehälter nach Fig. 1 in einer Seitenansicht mit einem Teilschnitt in der Wand des Behälters,
- Fig. 3: in einer stark vergrößerten unmaßstäblichen Darstellung einen Schnitt durch die Behälterwand gemäß dem in Fig. 2 eingezeichneten Teilschnitt,
- Fig. 4: in Form eines Blockschaltbildes ein Ablaufdiagramm für das erfindungsgemäße Verfahren zur Herstellung des Kunststoffbehälters nach den Figuren 1 und 2, und
- Fig. 5: ein Ausführungsbeispiel für eine vakuumgestützte Beschichtungsstation.

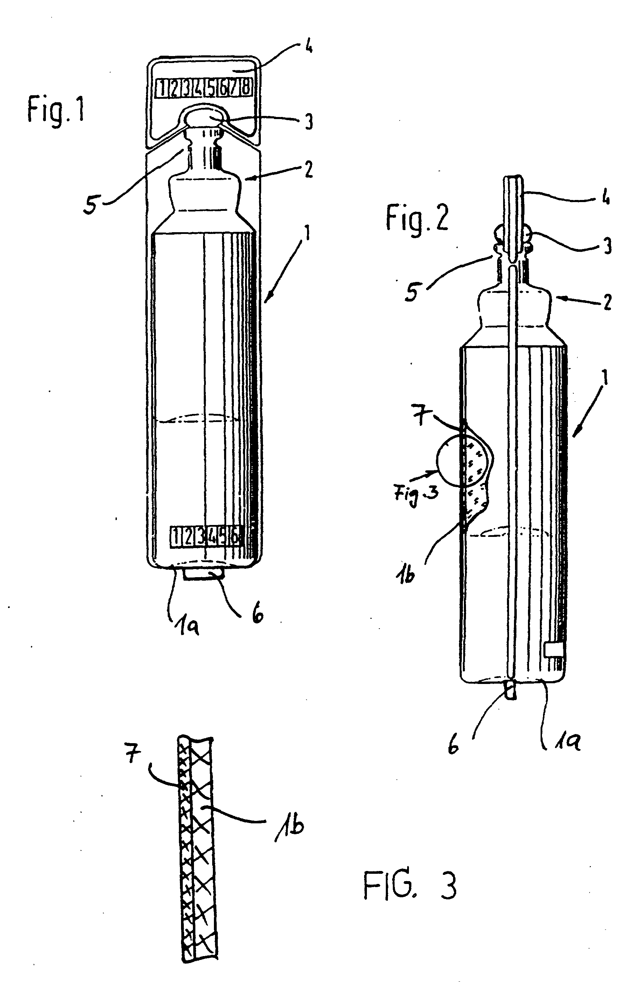

Die Fig. 1 zeigt eine durch Extrusionsblasen hergestellte formstabile Ampulle 1, die mit einer pharmazeutischen Flüssigkeit gefüllt ist. An den Hals 2 der Ampulle schließt sich ein einstückig mit ihm ausgebildeter Kopf 3 an, der seinerseits einstückig mit einem angeformten Knebel 4, der eine inhaltsspezifische Beschriftung trägt, ebenso wie am unteren Rand der Ampulle, ausgebildet ist.

Im oberen Teil des Halses 2 ist eine Sollbruchstelle 5 ausgeformt. Durch ein Verdrehen oder Kippen des Knebels 4 läßt sich somit im Applizierfall der Kopf 3 vom Hals 2 trennen, wodurch der Behälterinhalt freigegeben wird.

Der Behälter 1 weist ferner am Boden 1 a eine Anformung 6 auf, die als sogenannte "unkritische Stelle" als Angriffspunkt für Befestigungsmittel bei der Weiterbehandlung des verschlossenen Kunststoffbehälters dienen. Kopfseitig kann der Knebel 4 diese Halterungsfunktion übernehmen, da dieser Bereich ebenfalls "unkritisch" ist, da er vom Prinzip her nicht beschichtet werden muß.

Die befüllte Ampulle 1 nach den Figuren 1 und 2 ist in einem in-line-Verfahren, dem sogenannten "blow-fill-seal"-Verfahren, ausgeformt, befüllt und verschlossen worden. Dieses Verfahren ist - wie eingangs gewürdigt - Stand der Technik und braucht daher hier nicht näher beschrieben zu werden. Gemäß der Erfindung wird die befüllte und verschlossene Kunststoffampulle 1 gemäß den Figuren 1 und 2, ebenfalls im Rahmen der durchgängigen Fertigungslinie, mit einer äußeren Sperrschicht versehen, die so dünn ist, daß sie im Maßstab der Figuren 1 und 2 praktisch nicht darstellbar ist. Diese Sperrschicht 7 überdeckt nicht nur außen die Behälterwandung 1 b (einschließlich des Halses 2), wie der Teilschnitt in Fig. 2 und die vergrößerte Darstellung in Fig. 3 zeigt, sondern auch den Knebelverschluß 3, 4. Durch diese äußere Beschichtung der in-line hergestellten Ampulle im direkten Anschluß an deren Befüllung und Verschließen werden die eingangs beschriebenen Vorteile erzielt.

Die in den Figuren und 2 dargestellte Ampulle ist nur eine Ausführungsmöglichkeit für einen befüllten und verschlossenen Kunststoffbehälter. Dieser kann irgendeine, dem jeweiligen Zweck angepasste geometrische Form haben.

Als Kunststoffe für den Behälter 1 kommen vorzugsweise Polyethylen (PE), Polypropylen (PP), cyclische Olefine und deren Copolymere, Polybuten und Polymethylpenten in Frage, da diese Kunststoffe einer thermischen Sterilisierung des Kunststoffbehälters im Autoklaven widerstehen.

Die Wandstärke des Behälters 1 liegt dabei im mm-Bereich.

Das Material für die Sperrschicht 7 bestimmt sich nach dem Behältnisinhalt. Beispielsweise kommen Sperrschichten aus SiOₓC_{y}H_{z} und TiOₓC_{y}H_{z} in Frage. Eine besonders gute Sperrwirkung läßt sich erzielen, wenn eine derartige Sperrschicht schichtweise in unterschiedlichen Stöchiometrien aufgebaut ist.

Ebenso in Frage kommen Sperrschichten aus organischen Polymeren und aus anorganischen Oxiden, Nitriden und Oxinitriden. Auf die entsprechende Offenbarung in der eingangs zitierten DE 44 38 359 wird insoweit ausdrücklich Bezug genommen.

Die Sperrschicht kann ferner aus TiOₓC_{y}H_{z} oder aus Mischungen von TI/Si-Verbindungen bestehen.

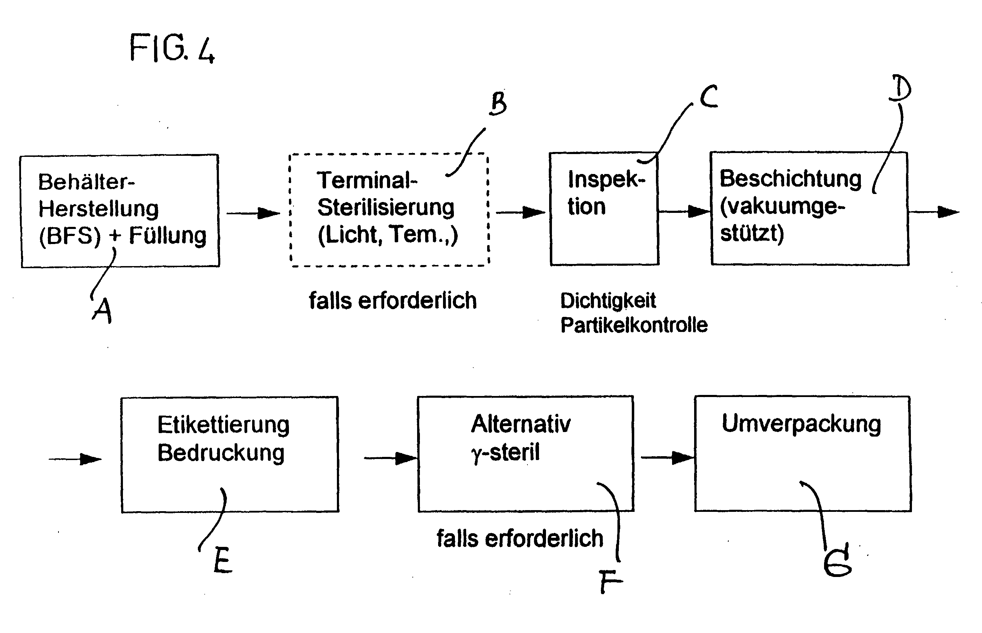

Die Dicke der Sperrschicht liegt im nm-Bereich, je nach Anwendung, und inhaltsspezifischen Anorderungen, insbesondere im Bereich von 20 bis 500 nm. Die Fig. 4 zeigt in einem Ablaufdiagramm die prinzipiellen Verfahrensschritte zum Herstellen des befüllten und verschlossenen sowie außen beschichteten Kunststoffbehälters, z.B. der Ampulle nach den Figuren 1, 2 bzw. 3.

Im Schritt A erfolgt die Herstellung des befüllten und verschlossenen Behälters, vorzugsweise in-line nach dem "blow-fill-seal"-Verfahren. Wie eingangs beschrieben, kann alternativ im Schritt A auch das "injection mold-fill-seal"-Verfahren auf der Basis der Ausformung des Kunststoffbehälters durch Spritzgießen angewendet werden. Im anschließenden - optionalen - Schritt B erfolgt eine Terminalsterilisation der bekannten Art, z.B. mit Licht oder durch Autoklavierung, z.B. bei 121° C für eine Dauer von 20 Minuten. Ob der optionale Schritt B im konkreten Fall durchgeführt wird, hängt typischerweise von dem Inhaltsstoff des Behälters ab, d.h. ob dieser die Sterilisation zuläßt.

Im Schritt C erfolgt eine Inspektion des befüllten und verschlossenen Kunststoffbehälters. Diese Inspektion bezieht sich insbesondere auf die Dichtigkeit (Leckprüfung) des Behälters und eine optische Kontrolle, hauptsächlich auf eine Partikel-Kontamination.

Im Anschluß daran erfolgt in dem Schritt D die Aufbringung der Sperrschicht auf den verschlossenen Kunststoffbehälter mittels eines vakuumgestützten Beschichtungsverfahrens, die dem Fachmann an sich geläufig sind und die auch in dem eingangs gewürdigten Stand der Technik an verschiedenen Stellen ausgeführt sind.

Die Anwendung derartiger vakuumgestützten Beschichtungsverfahren in Verbindung mit einem nach dem "blow-fill-seal"-Verfahren hergestellten nicht starren, d.h. deformierbaren Kunststoffbehälter, hat den Vorteil, daß bei der Beschichtung im Vakuum der Kunststoffbehälter etwas aufgebläht wird, der nach durchgeführtem Schritt D wieder schrumpft, so daß die Beschichtung unter eine Druckspannung gesetzt wird, die eine erhöhte mechanische Festigkeit bewirkt.

Bei der Beschichtung wird der Kunststoffbehälter oben und unten an "unkritischen Stellen" gehalten, d.h. Stellen, die keine Beschichtung aufweisen müssen, im Fall der Ampulle nach den Figuren 1 bis 3 an der Anformung 6 und dem Knebel 4.

Vorteilhaft für die Haftung ist es auch, wenn der zu beschichtende Kunststoffbehälter im Schritt D noch warm ist. Dies gelingt durch eine entsprechend hohe Temperaturführung im Schritt A.

Vorzugsweise erfolgt die Beschichtung aus einem Beschichtungsgas heraus mittels eines PECVD-Verfahrens (Plasma Enhanced Chemical Vapor Deposition), beispielsweise mittels eines Downstream-Plasmas unterstützten CVD-Verfahrens. Durch die Verwendung von Zusätzen von Sauerstoff oder Stickstoff sowie anderen Inert-Gasen zum Beschichtungsgas läßt sich eine ausreichende Haftung der Beschichtung auf dem Kunststoffmaterial des zu beschichtenden Behälters erzielen.

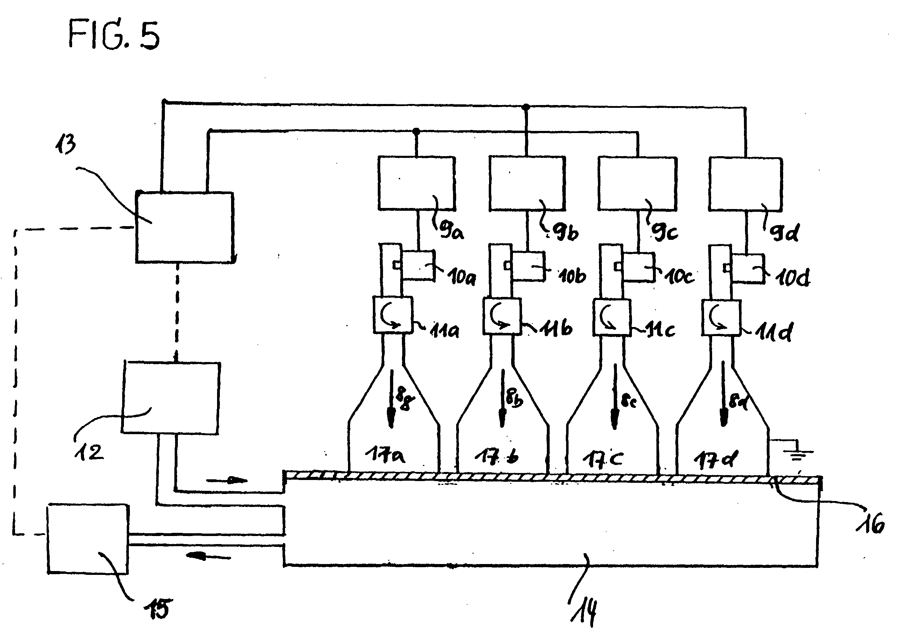

Der Kunststoffbehälter kann auch in einer Anlage entsprechend Fig. 5 nach dem PICVD-Verfahren beschichtet werden. Um nicht nur einen einzigen Behälter, sondern mehrere zusammenhängende Kunststoffbehälter gleichzeitig beschichten zu können, wird eine lineare Plasmaquelle in Kombination mit einem Durchlaufverfahren verwendet. Die plasmagestützten Beschichtungsverfahren ermöglichen es auch, daß das bei der Beschichtung auftretende Licht zur Reduktion der Keimzahl verwendet werden kann, d.h. der Schritt B kann mit dem Schritt D kombiniert sein.

Die Beschichtungsstation im Schritt D ist zweckmäßig als unabhängiges Modul konzipiert und greift nicht in den Abfüllraum des Schrittes A ein, der ja in einem Sterilbereich untergebracht sein muß. Die Beschichtung ist daher unabhängig von der Taktrate im Schritt A. Eine Prozeßführung, bei der innerhalb der Anlage des Schrittes A auch beschichtet würde, wäre aufgrund der relativ hohen Zykluszeiten der Beschichtung unwirtschaftlich, da die schnelle Taktzeit der "blow-fill-seal"-Anlage nicht ausgenutzt werden könnte.

Das Prinzip einer Durchlauf-Beschichtungsstation, in der das PICVD-Verfahren durchgeführt wird, ist in Fig. 5 dargestellt. In eine Vakuumkammer 14 werden fortlaufend die zu beschichtenden Behälter 1 nach Figuren 1 und 2 eingeführt (nicht dargestellt). Diese Vakuumkammer 14, die als Reaktionskammer dient, kann mittels einer Vakuumeinrichtung 15, die typischerweise eine Vakuumpumpe und die notwendigen Armaturen aufweist, evakuiert werden, z.B. auf einen Druck von 0,2 mbar. Oberhalb der Vakuumkammer 14, getrennt durch ein Mikrowellenfenster 16, befindet sich eine Reihe von Hom-Mikrowellenantennen 17 a bis 17 d, die eine lineare Plasmaquelle bilden. Über diese Mikrowellenantennen wird eine Mikrowellenstrahlung 8 a bis 8 d impulsweise in die Vakuumkammer 14 geleitet, die im Innern der Vakuumkammer ein Mikrowellenplasma bilden. Die Impulsdauer ist dabei ein zusätzlicher Parameter, der die Zusammensetzung der abgeschiedenen Schicht beeinflußt.

Die Mikrowellenimpulse, deren Dauer im Bereich von 0,1 bis 10 ms liegt, werden von Mikrowellengeneratoren 9 a bis 9 d erzeugt, die jeweils über ein Magnetron 10 a bis 10 d und Einwegleitungen 11 a bis 11 d mit den Mikrowellenantennen 17 a bis 17 d verbunden sind. Die Mikrowellenanordnung besitzt typischerweise Standard-Komponenten der 2,45 GHz-Technologie.

Über eine Gaszufuhr-Anordnung 12 wird einmal das Gas, in welchem das Plasma gezündet wird, typischerweise Sauerstoff und zum anderen das zur Schichtbildung notwendige Gas, das Reaktionsgas, eingeleitet. Im vorliegenden Ausführungsbeispiel sind es typischerweise metall-organische Reaktionsgase wie Siloxane, vorzugsweise Hexamethyldisiloxane (HMDSO), Tetramethyldisiloxan, Titantetraisopropoxide (TIPT) oder Silazane, aus denen über die Wahl einer geeigneten Impulsdauer die Schicht an der Außenseite des Behälters 1 (Fig. 3) aufgebaut wird.

Bei Beschichtungsprozessen, die eine erhöhte Substrattemperatur erfordern, kann bereits beim Befüllen im Schritt A das Befüllgut vorgewärmt werden, so daß eine Zusatzheizung auf die Prozeßtemperatur entfällt. Bei Prozessen mit Sterilisierung durch Hitze, kann definiert auf die Prozeßtemperatur gekühlt werden.

Eine Prozeßsteuerung 13 steuert den Ablauf in bekannter Weise, bei dem zunächst aus der Zufuhranordnung 12 die Mischung aus Sauerstoff und dem Reaktionsgas in die Vakuumkammer 14 eingeleitet wird. Danach wird jeweils durch einen Mikrowellenimpuls 8 a bis 8 d ein Plasma in der Vakuumkammer 14 gezündet, welches die Moleküle des Reaktionsgases cracked. Die dabei entstehenden Crack-Produkte diffundieren zur nächst gelegenen Oberfläche, - hier der zu beschichtenden Behälter - und formen nach und nach die gewünschte Schicht 7. In der Impulspause bis zur Zündung des nächsten Impulses, die im Bereich von 100 ms liegt, werden jeweils die verbrauchten Reaktionsgase aus der Vakuumkammer nach Art eines Zweitaktmotors durch Absaugen mittels der Vakuumstufe 15 entfernt und durch frisches Reaktionsgas und Sauerstoff ersetzt.

Grundsätzlich kann auch eine Mehrfachschicht erzeugt werden. Dazu wird, sobald die genügende Schichtdicke für die erste Schicht erreicht ist, das zugehörige Reaktionsgas durch das zur Erzeugung der zweiten Schicht notwendige Reaktionsgas ersetzt. Zur Erzeugung eines unscharfen Überganges zwischen den beiden Schichten kann für einen gewissen Zeitraum ein Gemisch aus beiden Reaktionsgasen eingeleitet werden. Für gleichmäßige Übergänge kann man dabei den Anteil des ersten Reaktionsgases vermindern und gleichzeitig den Anteil des zweiten Reaktionsgases stetig bis auf den Nennwert erhöhen.

Die PICVD-Technologie (Plasma-Impuls-Chemical-Vapor-Deposition) ist beispielsweise bekannt durch die DE 40 08 405 C1 sowie durch die US-A-5,154,943 und wird bislang insbesondere zur Erzeugung von Sperrschichten auf Kunststoffbehältern angewendet (DE 44 38 359 A1).

Die lineare Plasmaquelle nach Fig. 5 ist grundsätzlich durch die DE 38 30 249 C2 bekannt geworden, wobei die Steuerung der einzelnen Mikrowellenantennen 8 a bis 8 d vorzugsweise durch das zeitversetzte Betreiben zweier benachbarter Mikrowellenantennen gemäß der älteren Patentanmeldung 196 34 795.5 erfolgt.

Zusätzlich oder anstelle von Sauerstoff als Gas für das Plasma können auch andere aus der Plasmatechnik bekannte Gase zur Erzeugung eines Plasmas verwendet werden, wie beispielsweise Argon, Helium, Wasserstoff oder Stickstoff; weitere Beispiele dafür werden z.B. in dem Buch "Plasma-Technik" von Schade u.a., erschienen 1990 im Verlag Technik GmbH Berlin, beschrieben.

Nach dem Auftragen der Außenschicht im Schritt D erfolgt das Etikettieren/Bedrucken des beschichteten Kunststoffbehälters (Schritt E). Danach erfolgt, falls erforderlich, eine Gamma-Sterilisation, falls der Schritt B nicht durchgeführt werden konnte (Schritt F). Am Ende des Prozesses steht die Verpackung der Behälter in eine Umverpackung für den Transport zum Empfänger (Schritt G).

## Patentansprüche

1. Befüllter und verschlossener Kunststoffbehälter (1), ausgeformt nach der Extrusionsblas-, Spritzblas- oder Spritzgießtechnik, und in-line befüllt sowie verschlossen, der mindestens eine Sperrschicht (7) gegen Gase, Wasserdampf oder organische Substanzen aufweist, dadurch gekennzeichnet, daß die Sperrschicht (7) aus einem behälterinhaltsspezifischen Material besteht und an der Außenseite des befüllten Kunststoffbehälters (1), einschließlich dessen Verschlusses (3, 4) nach seiner Befüllung aufgebracht ist.

2. Kunststoffbehälter nach Anspruch 1, dadurch gekennzeichnet, daß der zu befüllende und zu verschließende Behälter (1) ein nicht starrer Behälter ist.

3. Kunststoffbehälter nach Anspruch 1 oder 2, dadurch gekennzeichnet, daß er nach dem "Blow-Fill-Seal"-Verfahren hergestellt ist.

4. Kunststoffbehälter nach einem der Ansprüche 1 bis 3, dadurch gekennzeichnet, daß das Material des Kunststoffbehälters (1) Polyethylen oder Polypropylen oder Copolymere davon sind.

5. Kunststoffbehälter nach einem der Ansprüche 1 bis 4, dadurch gekennzeichnet, daß die Sperrschicht (7) aus SiOₓC_{y}H_{z}, oder TiOₓC_{y}H_{z} oder aus Mischungen von Si- und Ti-Verbindungen besteht.

6. Kunststoffbehälter nach Anspruch 5, dadurch gekennzeichnet, daß die Sperrschicht (7) aus SiOₓC_{y}H_{z} schichtweise in unterschiedlichen Stöchiometrien aufgebaut ist.

7. Verfahren zum Herstellen eines befüllten, verschlossenen und beschichteten Kunststoffbehälters nach Anspruch 1 oder einem der folgenden, mit den in einer geschlossenen Fertigungslinie ausgeübten Schritten:
- Ausformen des Kunststoffbehälters,
- Befüllen des Kunststoffbehälters,
- Verschließen des Kunststoffbehälters,
- optional Sterilisieren des verschlossenen Kunststoffbehälters
- Beschichten der Außenseite des Kunststoffbehälters einschließlich seines Verschlusses nach seiner Befüllung mit einer behälterinhaltsspezifischen Sperrschicht, und
- optional Sterilisieren des beschichteten Kunststoffbehälters.

8. Verfahren nach Anspruch 7, dadurch gekennzeichnet, daß das Beschichten mittels eines Vakuumverfahrens, vorzugsweise dem PECVD-Verfahren, erfolgt.

9. Verfahren nach Anspruch 8, dadurch gekennzeichnet, daß das Vakuumverfahren ein vorzugsweise mittels eines Downstream-Plasma unterstütztes CVD-Verfahren ist.

10. Verfahren nach Anspruch 8, dadurch gekennzeichnet, daß das Vakuumverfahren ein PICVD-Verfahren mit einer linearen Plasmaquelle und einem kontinuierlichen Durchlauf ist.

11. Verfahren nach Anspruch 9 oder 10, dadurch gekennzeichnet, daß das Licht des Plasmas zugleich zur Keimzahlreduktion des Behälterinhaltes verwendet wird.

12. Verfahren nach einem der Ansprüche 7 bis 11, dadurch gekennzeichnet, daß die Beschichtung auf die vom Ausformvorgang des Kunststoffbehälters noch warme Behälteroberfläche erfolgt.

13. Verfahren nach einem der Ansprüche 7 bis 11, dadurch gekennzeichnet, daß das Füllgut des Kunststoffbehälters vorgewärmt ist, um eine definierte Temperatur beim Beschichten zu erhalten.

## Claims

1. Filled and closed plastic container (1), formed by the extrusion blow-moulding, injection blow-moulding or injection-moulding technique, and filled and closed in-line, which has at least one barrier layer (7) resisting gases, water vapour or organic substances, characterized in that the barrier layer (7) consists of a material specific to the container content and is applied on the outer side of the filled plastic container (1), including the closure (3, 4) of the latter, after it has been filled.

2. Plastic container according to Claim 1, characterized in that the container (1) to be filled and closed is a non-rigid container.

3. Plastic container according to Claim 1 or 2, characterized in that it is manufactured by the "blow-fill-seal" method.

4. Plastic container according to one of Claims 1 to 3, characterized in that the material of the plastic container (1) is polyethylene or polypropylene or copolymers thereof.

5. Plastic container according to one of Claims 1 to 4, characterized in that the barrier layer (7) consists of SiOₓCyH_{z}, or TiOₓC_{y}H_{z} or of mixtures of Si and Ti compounds.

6. Plastic container according to Claim 5, characterized in that the barrier layer (7) is made up from SiOₓCyH_{z} layer by layer in different stoichiometries.

7. Process for manufacturing a filled, closed and coated plastic container according to Claim 1 or one of the following claims, with the steps performed in a closed production line:
- forming the plastic container,
- filling the plastic container,
- closing the plastic container,
- optionally, sterilizing the closed plastic container,
- coating the outer side of the plastic container, including its closure, after it has been filled, with a barrier layer specific to the container content, and
- optionally, sterilizing the coated plastic container.

8. Process according to Claim 7, characterized in that the coating is performed by means of a vacuum method, preferably the PECVD method.

9. Process according to Claim 8, characterized in that the vacuum method is a CVD method preferably assisted by means of a downstream plasma.

10. Process according to Claim 8, characterized in that the vacuum method is a PICVD method with a linear plasma source and a continuous throughput.

11. Process according to Claim 9 or 10, characterized in that the light of the plasma is used at the same time for reducing the bacterial count of the container content.

12. Process according to one of Claims 7 to 11, characterized in that the coating is carried out on the container surface still warm from the operation of forming the plastic container.

13. Process according to one of Claims 7 to 11, characterized in that the product with which the plastic container is filled is pre-heated, in order to obtain a defined temperature during the coating.

## Revendications

1. Récipient de matière synthétique rempli et obturé (1), formé selon la technique de soufflage par extrusion, de soufflage par injection ou de moulage par injection, et rempli et obturé en ligne, qui présente au moins une couche barrière (7) contre les gaz, la vapeur d'eau et les substances organiques, caractérisé en ce que la couche barrière (7) est constituée d'un matériau spécifique au contenu du récipient et est appliquée sur la partie extérieure du récipient en matière synthétique rempli (1), y compris de son obturateur (3, 4), après son remplissage.

2. Récipient de matière synthétique selon la revendication 1, caractérisé en ce que le récipient à remplir et à fermer (1) est un récipient non rigide.

3. Récipient de matière synthétique selon la revendication 1 ou 2, caractérisé en ce qu'il est fabriqué selon le procédé "Blow-Fill-Seal".

4. Récipient de matière synthétique selon l'une quelconque des revendications 1 à 3, caractérisé en ce que le matériau du récipient de matière synthétique (1) est constitué de polyéthylène ou de polypropylène ou de copolymères de ceux-ci.

5. Récipient de matière synthétique selon l'une quelconque des revendications 1 à 4, caractérisé en ce que la couche barrière (7) est constituée de SiOₓC_{y}H_{z} ou de TiOₓC_{y}H_{z} ou encore de mélanges de composés de Si et de Ti.

6. Récipient de matière synthétique selon la revendication 5, caractérisé en ce que la couche barrière (7) est constituée de SiOₓC_{y}H_{z} en feuilles selon différentes stoechiométries.

7. Procédé de fabrication d'un récipient de matière synthétique rempli, obturé et revêtu selon la revendication 1 ou selon l'une des étapes suivantes effectuées dans une chaîne de production fermée :
- formation du récipient de matière synthétique,
- remplissage du récipient de matière synthétique,
- obturation du récipient de matière synthétique,
- stérilisation facultative du récipient de matière synthétique obturé,
- revêtement de la partie extérieure du récipient de matière synthétique, y compris de son obturateur après son remplissage, par une couche barrière spécifique au contenu du récipient, et
- stérilisation facultative du récipient de matière synthétique revêtu.

8. Procédé selon la revendication 7, caractérisé en ce que le revêtement se fait au moyen d'un procédé sous vide, de préférence le procédé PECVD.

9. Procédé selon la revendication 8, caractérisé en ce que le procédé sous vide est un procédé CVD soutenu de préférence par un plasma downstream.

10. Procédé selon la revendication 8, caractérisé en ce que le procédé sous vide est un procédé PICVD avec une source de plasma linéaire et un défilement en continu.

11. Procédé selon la revendication 9 ou 10, caractérisé en ce que la lumière du plasma est utilisée simultanément pour la réduction du nombre de germes dans le contenu du récipient.

12. Procédé selon l'une quelconque des revendications 7 à 11, caractérisé en ce que le revêtement se fait sur la surface encore chaude du récipient après l'opération de formation du récipient de matière synthétique.

13. Procédé selon l'une quelconque des revendications 7 à 11, caractérisé en ce que l'article de remplissage du récipient de matière synthétique est préchauffé pour maintenir une température définie lors du revêtement.
